# EUROPEAN PATENT SPECIFICATION

(11) **EP 0 328 380 B1**
(45) Date of publication and mention of the grant of the patent: **12.01.1994**
(21) Application number: 89301237.7
(22) Date of filing: 09.02.1989
(51) Int. Cl.: G01N 27/00, G01N 33/53

(54) **Electrochemical sensors in immunological measurement**
Elektrochemische Sensoren für immunologische Testverfahren
Capteurs électrochimiques pour essai immunologique

(30) Priority: 10.02.1988 JP 27471/88
(43) Date of publication of application: 16.08.1989
(73) Proprietor: NEC CORPORATION, Tokyo (JP)
(72) Inventor: Kuriyama, Toshihide c/o NEC Corporation, Minato-ku Tokyo (JP)
(74) Representative: Moir, Michael Christopher

(56) References cited:
- EP-A- 0 127 438
- EP-A- 0 155 193
- CHEMICAL ABSTRACTS, vol. 90, no. 1, 1st January 1979, page 244, abstract no. 2418z, Columbus, Ohio, US; Y.K. CHO et al.: "Immobilization of enzymes on activated carbon: properties of immobilized glucoamylase, glucose oxidase, and gluconolactonase", & BIOTECHNOL. BIOENG. 1978, 20(10), 1651-65
- CHEMICAL ABSTRACTS, vol. 110, no. 19, 8th May 1989, page 667, abstract no. 17859z, Columbus, Ohio, US; H. YOSHIO et al.: "Glucose-sensitive field-effect transistor with a membrane containing coimmobilized gluconolactonase and glucose oxidase", & ANAL. CHIM. ACTA 1988, 212(1-2), 49-59

## Description

### Background of the Invention

### Field of the Invention

The present invention relates to electrochemical sensors used in immunological measurement for determining a very small amount of an antigen or an antibody, more particularly it relates to an electrochemical immunochemical sensor system which employes an ion selective electrode or an ion sensitive field effect transistor (ISFET) as a detector electrode in enzyme immunoassay.

### Description of the Related Art

Enzyme immunoassay (hereinafter, EIA) which utilizes the specificity of antigen-antibody reaction is one of well-known techniques for determining a very small amount of antigen or antibody. The principle of the EIA is as follow:

In a sandwich assay, an antigen or an antibody to be measured is reacted with a fixed antigen or antibody which has a specific reactivity with the antigen or antibody to be measured. After non-reacted antigen or antibody is swept, the bound antigen or antibody is further reacted with enzyme-labeled antigen or antibody which has a specific reactivity or affinity with the antigen or antibody to be measured. After non-reacted antigen or antibody is swept, the proportion of the conjugated enzyme-labeled antigen or antibody is measured by an enzyme reaction, so that the quantity of the antigen or antibody to be measured is determined.

In a competitive assay, a predetermined amount of labeled antigen or labeled antibody is added to a solution containing an antigen or an antibody to be measured. Then, a reaction with a fixed antigen or antibody which has a specific reactivity with the antigen or antibody to be measured is effected. After non-reacted antigen or antibody is swept, the proportion of the conjugated enzyme-labeled antigen or antibody is measured by an enzyme reaction, so that the quantity of the antigen or antibody to be measured is determined.

In the above-mentioned techniques of EIA, the enzyme activity or the proportion of the conjugated enzyme-labeled antigen or antibody can be determined by measuring the absorbance or the fluorescence of a substrate or the enzymic reaction product, by measuring the quantitative change of dissolved oxygen by means of an oxygen electrode or by measuring the pH change by means of an ion selective electrode or the like.

When the enzyme activity is determined by the pH change, an ion sensitive field effect transistor (hereinafter ISFET) can be used as a pH electrode. The ISFET is expected to be a high sensitive sensor or probe for measuring a very small amount of samples precisely in the field of immunological assay.

However, the ion selective electrode or ISFET electrode can not be applied to practical uses in an immunochemical measurement method in which glucose oxidase is used as a labeling enzyme. In fact, when a detector electrode on which an antigen or an antibody labeled with glucose oxidase is attached by an immuno-reaction is immersed in a glucose solution whose enzyme activity is to be determined and whose glucose will be oxidized with glucose oxidase, the pH change does not proceed quickly, in other words the variation in pH value is very small, so that it is difficult to determine the amount of labeling enzyme satisfactorily.

Examples of prior art immunoassay techniques are disclosed in:
(1) "Electrochemical Sensors in Immunological Analysis" edited by T.T.Ngo, Plenum Press, 1987, p. 239-255.
(2) Anal. Chem. 1985, 57,2754-2756
An object of the present invention is to overcome the problems in the prior art and to provide a highly-sensitive sensor system which can accelerate the measuring speed and to increase the variation in pH when an ion selective electrode or an ISFET electrode is used as a detector electrode in the enzyme immunoassay.

### SUMMARY OF THE INVENTION

An electrochemical sensor system for determining the quantity of an antigen or an antibody by the enzyme immunoassay according to the present invention includes an electrode for detecting a change in electrochemical value and at least one enzyme labeled antigen or antibody which can be bound to an antigen or antibody to be measured, characterised in that said enzyme labeled antigen or antibody is labeled with at least the two enzymes glucose oxidase and gluconolactonase, and in that said electrode is a hydrogen ion selective electrode or a hydrogen ion sensitive field effect transistor (ISFET).

The enzyme immunoassay according to the present invention can be carried out by any one of the conventional techniques including a sandwich assay or a competitive assay.

In the case of sandwich assay or competitive assay, it is used usually with a fixed antigen or antibody which is fixed to the electrode and which has a specific reactivity with the antigen or antibody to be measured in case of the sandwich assay or with the enzyme-labeled antigen or antibody in the case of the competitive assay.

According to a preferred embodiment of the present invention, the antigen or antibody which is labeled with glucose oxidase and gluconolactonase are attached on a surface of a detector electrode by an immuno-reaction and then is immersed in a glucose solution whose enzyme activity is to be determined. In the glucose solution, the following reactions proceed: 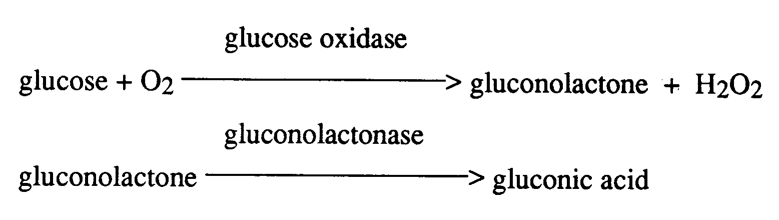
This means that, in the conventional method in which the antigen or antibody is labeled with glucose oxidase alone, it takes a long time until glucose is converted to gluconic acid because glucose is oxidized with glucose oxidase in the first step and then the resulting gluconolactone is slowly hydrolyzed without gluconolactonase into gluconic acid formation of which is detectable by an ion selective electrode or an ISFET, so that the pH change does not occur quickly and hence a big output signal can not be obtained.

To the contrary, according to the present invention in which an antigen or antibody labeled with glucose oxidase and gluconolactonase is used, the gluconolactone which is an oxidation product of glucose is immediately hydrolyzed with gluconolactonase into gluconic acid, so that a big pH change occur and hence a significant output signal which is detectable by the ion selective electrode or ISFET is obtained.

According to the present, higher sensitivity in measurement is assured by the enzyme immuno-sensors which use potentiometric detector electrodes. In fact, when glucose is used as a substrate in the potentiometric enzyme immuno-sensors, if the antigen or antibody labelled with only glucose oxidase as is the conventional amperometric immuno-sensors, a big output signal can not be obtained because of such a fact that glucose is oxidized in the first step with glucose oxidase which is immobilized on a surface of a detector electrode by the immuno-reaction and then it takes a long time until gluconolactone is converted into gluconic acid which is detectable by an ion selective electrode or an ISFET.

To the contrary, according to the present invention, both of glucose oxidase and gluconolactonase are labeled simultaneously. Therefore, in addition to glucose oxidase, gluconolactonase is also immobilized by the immuno-reaction on the surface of the detector electrode and the gluconolactone which is an oxidation product of glucose is immediately hydrolyzed with gluconolactonase into gluconic acid, so that a big pH change occur in a short time and hence the highly sensitive measurement is assured even by the ion selective electrode or ISFET.

Now, an example of the present invention will be described in more details with reference to attached drawings.

### Brief Description of the Drawings

Fig. 1A, 1B and 1C illustrate three steps for carrying out the measuring method according to the present invention for determining human IgG by an ISFET on which surface the sheep anti-human IgG is immobilized.

Fig. 2 shows the response characteristics when the ISFET which is subjected to an immuno-reaction is immersed in a glucose solution.

Fig. 3 shows a relation between the output of ISFET and the concentration of human IgG.

### Example

In this Example, human immuno globuline G (hereinafter human IgG) is determined.

At first, 5 mg of glucose oxidase and 10 mg of gluconolactonase are dissolved in 0.3 ml of 0.2 M phosphate buffer (pH 6.8) containing 1.25 % of glutaraldehyde and are left at ambient temperature for 20 hours. Then, the solution is applied to Sephadex column equilibrated with 0.15 M sodium chloride (NaCl) and eluted. 1 ml of the resulting eluted solution of glucose oxidase and gluconolactonase is mixed with 1.0 ml of 0.15 M NaCl solution containing 5 mg of sheep anti-human IgG and 0.1 ml of 1 M sodium carbonate buffer (pH 9.5) and is left for 2 hours at 4 °C. The mixed solution is dialyzed against buffered physiological saline to obtain a sheep anti-human IgG labeled with glucose oxidase and gluconolactonase.

By using this sheep anti-human IgG labeled with glucose oxidase and gluconolactonase, the concentration of human IgG is determined as follow:

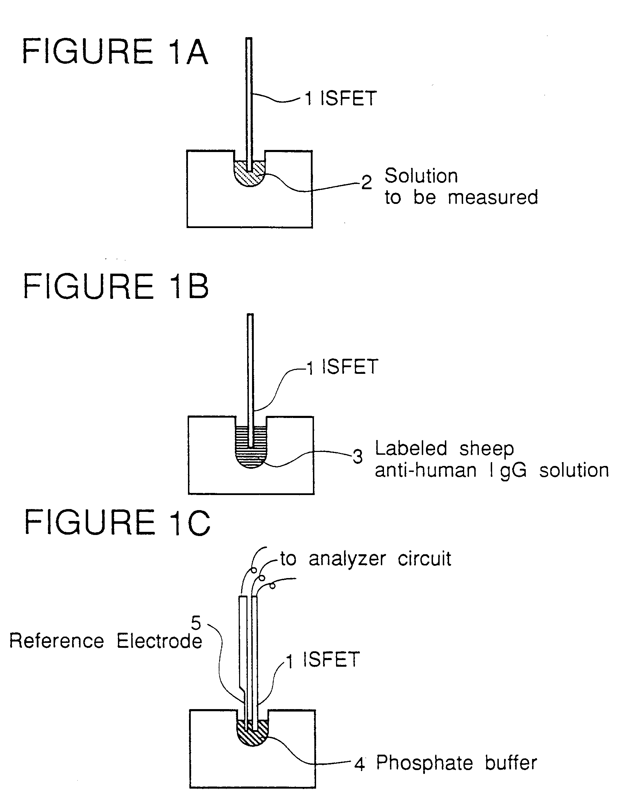

At first, as is shown in Fig. 1A, an ISFET (1) on a surface of which sheep anti-human IgG is immobilized is immersed in a solution (2) to be measured and containing human IgG for 5 minutes to perform antigen-antibody reaction. Then, the ISFET (1) is immersed in the solution (3) of sheep anti-human IgG labeled with glucose oxidase and gluconolactonase for 5 minutes as is shown in Fig. 1B. After then, as is shown in Fig. 1C, the ISFET (1) is immersed in 0.01 mM phosphate buffer (4) (pH 6.8) the glucose concentration of which is 500 mg/dl to measure the pH change in a membrane fixed on the surface of the ISFET (1).

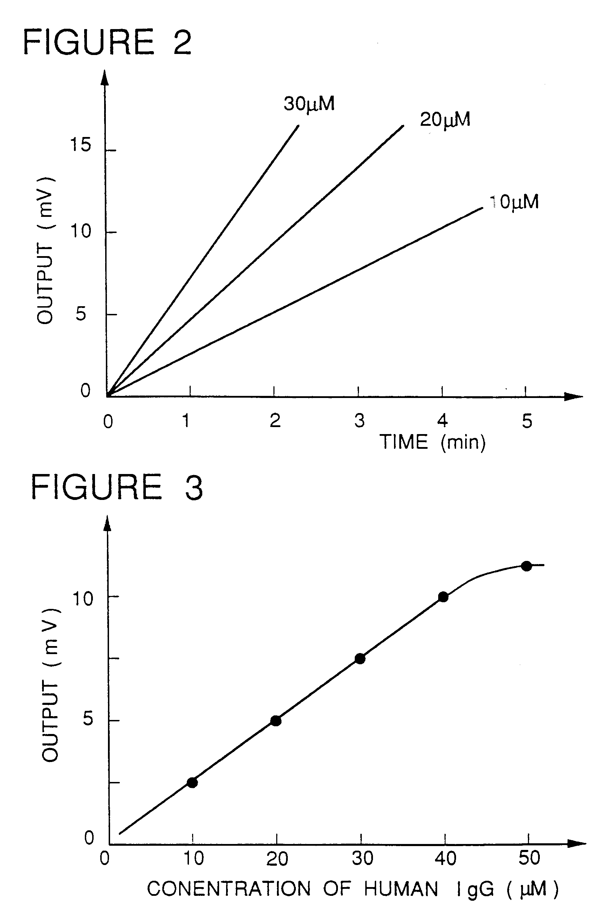

Fig. 2 shows response characteristics for the respective concentrations of the human IgG of 10 µM, 20 µM and 30 µM, when the ISFET is immersed in the glucose solution after the immuno-reaction. The output signal of ISFET is recorded by a source follower circuit. Fig. 2 reveals such a fact that the output level increase with time elapsed.

Fig. 3 shows a relation between the output of ISFET and the concentration of human IgG in one minute after the ISFET is immersed in the glucose solution.

It is apparent from the Example that a big output signal can be obtained in a short time after the immune-reacted ISFET is immersed in the glucose solution according to the present invention in which both of glucose oxidase and gluconolactonase are used as labeling enzymes, so that higher sensitivity of measurement is assured.

Although glucose oxidase and gluconolactonase are reacted simultaneously with the antibody to obtain the labelled antibody in the Example, it is also possible to react the antigen with glucose oxidase at first and then with gluconolactonase separately.

## Claims

1. An electrochemical sensor system for determining the quantity of an antigen or an antibody by enzyme immunoassay, including an electrode for detecting a change in electrochemical value and at least one enzyme labeled antigen or antibody which can be bound to an antigen or antibody to be measured, characterised in that said enzyme labeled antigen or antibody is labeled with at least the two enzymes glucose oxidase and gluconolactonase, and in that said electrode is a hydrogen ion selective electrode or a hydrogen ion sensitive field effect transistor.

2. The electrochemical sensor system set forth in Claim 1, characterised in that the enzyme immunoassay is carried out by a sandwich assay or a competitive assay.

3. The electrochemical sensor system set forth in Claim 1 or 2, characterised by including further an antigen or antibody which is fixed to the electrode and which has a specific reactivity with the antigen or antibody to be measured in case of the sandwich assay or with the enzyme labeled antigen or antibody in the case of the competitive assay.

4. The electrochemical sensor system set forth in any one of Claims 1 to 3, characterised in that said antigen or antibody to be measured is human IgG.

5. Immunological measurement method by enzyme immunoassay for determining the quantity of an antigen or an antibody by an electrochemical sensor, characterised by using an enzyme-labeled antigen or antibody labeled with at least the two enzymes glucose oxidase and gluconolactonase and by determining the quantity of the antigen or antibody with a hydrogen ion selective electrode or a hydrogen ion sensitive field effect transistor.

6. The immunological measurement method set forth in Claim 5, characterised in that the enzyme immunoassay is carried out by a sandwich assay or a competitive assay.

7. The immunological measurement method set forth in Claim 5 or 6, characterised in that the antigen or antibody to be measured or the enzyme labeled antigen or antibody is bound to an antigen or antibody which is fixed to the electrode and which has a specific reactivity to them.

8. The immunological measurement method set forth in any one of Claims 5 to 7, characterised in that said antigen or antibody to be measured is human IgG.

## Patentansprüche

1. Elektrochemisches Sensorsystem zur Bestimmung der Menge eines Antigens oder Antikörpers durch Enzymimmunoassay mit einer Elektrode zur Messung der Veränderung einer elektrochemischen Größe und mit mindestens einem enzymmarkierten Antigen oder Antikörper, das bzw. der an ein zu bestimmendes Antigen bzw. einen Antikörper gebunden sein kann, dadurch gekennzeichnet, daß das enzymmarkierte Antigen bzw. der Antikörper mit mindestens den zwei Enzymen Glucoseoxidase und Gluconolactonase markiert ist, und daß die Elektrode eine Wasserstoffionen-selektive Elektrode oder ein Wasserstoffionen-empfindlicher Feldeffekttransistor ist.

2. Elektrochemisches Sensorsystem nach Anspruch 1, dadurch gekennzeichnet, daß der Enzymimmunoassay mittels eines Sandwichassays oder eines kompetitiven Assays durchgeführt wird.

3. Elektrochemisches Sensorsystem nach Anspruch 1 oder 2, dadurch gekennzeichnet, daß es weiterhin ein Antigen oder einen Antikörper umfaßt, das bzw. der auf der Elektrode befestigt ist und eine spezifische Reaktivität mit dem zu bestimmenden Antigen oder Antikörper im Falle des Sandwichassays oder mit dem enzymmarkierten Antigen oder Antikörper im Falle des kompetitiven Assays aufweist.

4. Elektrochemisches Sensorsystem nach einem jeden der Ansprüche 1 bis 3, dadurch gekennzeichnet, daß das zu bestimmende Antigen bzw. der Antikörper Human-IgG ist.

5. Immunologisches Meßverfahren mittels Enzymimmunoassay zur Bestimmung der Menge eines Antigens oder Antikörpers mit einem elektrochemischen Sensor, gekennzeichnet durch die Verwendung eines enzymmarkierten Antigens oder Antikörpers, markiert mit mindestens den zwei Enzymen Glucoseoxidase und Gluconolactonase, sowie durch die Bestimmung der Menge des Antigens oder Antikörpers mit einer Wasserstoffionen-selektiven Elektrode oder eines Wasserstoffionen-empfindlichen Feldeffekttransistors.

6. Immunologisches Meßverfahren nach Anspruch 5, dadurch gekennzeichnet, daß der Enzymimmunoassay mittels eines Sandwichassays oder eines kompetitiven Assays durchgeführt wird.

7. Immunologisches Meßverfahren gemäß Anspruch 5 oder 6, dadurch gekennzeichnet, daß das zu bestimmende Antigen bzw. der Antikörper oder das enzymmarkierte Antigen bzw. der Antikörper an ein Antigen bzw. einen Antikörper gebunden sind, daß bzw. der auf der Elektrode befestigt ist und gegenüber diesen eine spezifische Reaktivität aufweist.

8. Immunologisches Meßverfahren gemäß einem jeden der Ansprüche 5 bis 7, dadurch gekennzeichnet, daß das zu bestimmende Antigen bzw. der Antikörper Human-IgG ist.

## Revendications

1. Système de capteur électrochimique pour déterminer la quantité d'un antigène ou d'un anticorps par dosage immunologique par enzyme, comprenant une électrode pour détecter un changement de la valeur électrochimique et au moins un antigène ou un anticorps marqué par enzyme qui peut être lié à un antigène ou à un anticorps devant être mesuré, caractérisé en ce que ledit antigène ou ledit anticorps marqué par enzyme est marqué avec au moins les deux enzymes glucose oxydase et gluconolactonase, et en ce que ladite électrode est une électrode sélective vis-à-vis de l'ion hydrogène ou un transistor à effet de champ sensible à l'ion hydrogène.

2. Système de capteur électrochimique selon la revendication 1, caractérisé en ce que le dosage immunologique par enzyme est exécuté par un dosage en sandwich ou un dosage compétitif.

3. Système de capteur électrochimique selon la revendication 1 ou 2, caractérisé en ce qu'il comprend en outre un antigène ou un anticorps qui est fixé à l'électrode et qui présente une réactivité spécifique avec l'antigène ou l'anticorps à mesurer dans le cas du dosage en sandwich ou avec l'antigène ou l'anticorps marqué par enzyme dans le cas du dosage compétitif.

4. Système de capteur électrochimique selon l'une quelconque des revendications 1 à 3, caractérisé en ce que ledit antigène ou ledit anticorps à mesurer est l'IgG de l'être humain.

5. Procédé de mesure immunologique par dosage immunologique par enzyme pour déterminer la quantité d'un antigène ou d'un anticorps par un capteur électrochimique, caractérisé par l'utilisation d'un antigène marqué par enzyme ou d'un anticorps marqué par au moins les deux enzymes glucose oxydase et gluconolactonase et par la détermination de l'antigène ou de l'anticorps avec une électrode sélective vis-à-vis de l'ion hydrogène ou un transistor à effet de champ sensible à l'ion hydrogène.

6. Procédé de mesure immunologique selon la revendication 5, caractérisé en ce que le dosage immunologique par enzyme est exécuté par un dosage en sandwich ou un dosage compétitif.

7. Procédé de mesure immunologique selon la revendication 5 ou 6, caractérisé en ce que l'antigène ou l'anticorps à mesurer ou l'antigène ou l'anticorps marqué par enzyme est lié à un antigène ou à un anticorps qui est fixé à l'électrode et qui présente une réactivité spécifique vis-à-vis d'eux.

8. Procédé de mesure immunologique selon l'une quelconque des revendications 5 à 7, caractérisé en ce que ledit antigène ou ledit anticorps à mesurer est l'IgG de l'être humain.
